# EUROPEAN PATENT APPLICATION

(11) **EP 4 371 493 A1**
(43) Date of publication of application: **22.05.2024**
(21) Application number: 22953378.1
(22) Date of filing: 18.08.2022
(51) Int. Cl.: A61B 5/346, A61B 5/00, G16H 50/20, G16H 50/70, G16H 40/20

(54) **METHOD FOR ECG READING SERVICE PROVIDING**

(71) Applicant: Medicalai Co., Ltd., Seoul 06302 (KR)
(72) Inventor: KWON, Joon Myoung, Seoul 06629 (KR)
(74) Representative: Isarpatent
(86) International application number: PCT/KR2022/012368
(87) International publication number: WO 2024/038932

(57) **Abstract**

The present invention is directed to a method of providing electrocardiogram reading service. According to the present invention, there is provided a method of providing electrocardiogram reading service by using a system for providing electrocardiogram reading service, the method including: receiving an electrocardiogram reading request signal and electrocardiogram data from a client terminal; outputting primary electrocardiogram reading data by inputting the received electrocardiogram data to an electrocardiogram reading model in response to the received electrocardiogram reading request signal; when receiving an additional electrocardiogram request signal from the client, acquiring secondary electrocardiogram reading data by transmitting the primary electrocardiogram reading data to a medical person terminal including a designated medical specialist; calculating a fee in accordance with an electrocardiogram reading service provision range; and transmitting the primary electrocardiogram reading data or secondary electrocardiogram reading data and the calculated fee to the client terminal.

## Description

### Technical Field

The present invention relates to a method of providing electrocardiogram reading service, and more particularly to a method of providing electrocardiogram reading service by which primary reading results are acquired from electrocardiogram data received from a client through an artificial intelligence algorithm and final reading results are provided by transmitting the electrocardiogram data and the primary reading results to medical staff.

### Background Art

An electrocardiogram is a graphical record of electrical potentials related to heartbeat on the surface of the human body. Such electrocardiograms include not only standard 12-lead electrocardiograms but also exercise stress electrocardiograms and Holter electrocardiograms. Electrocardiograms are used for the examination and diagnosis of circulatory diseases, and have the advantages of being simple, relatively inexpensive, non-invasive, and easily recorded repeatedly.

As for a standard 12-lead electrocardiogram used in hospitals, six electrodes are attached to the front of the chest, three electrodes (four electrodes when a ground electrode is included) are attached to the limbs, 12-lead information is all collected and combined, and then a disease is diagnosed. A 12-lead electrocardiogram records electrical potentials of the heart in 12 electrical directions centered on the heart. Through this, the disease of the heart confined to one area can be accurately diagnosed.

Electrocardiography is widely used for general treatment, basic examinations for hospitalization and surgery, and health checkups in that it is an important test that can diagnose serious diseases such as myocardial infarction and arrhythmia and is also an inexpensive, non-invasive, rapid and easy test that takes 10 seconds to check basic heart disease conditions.

However, the number of doctors who can accurately read electrocardiograms is limited. Electrocardiogram reading ability is not simply learned through books, but is learned experientially by treating patients with heart disease, measuring electrocardiograms, and performing various confirmatory tests such as actual echocardiography, cardiovascular angiography, and blood tests. Accordingly, it is difficult to accurately read electrocardiograms unless a person is a cardiologist, thoracic surgeon, or emergency medical staff member who continually examines patients with heart disease.

Recently, electrocardiograms have been read using automatic reading algorithms. However, due to the nature of rule-based reading, accuracy may be reduced because of an external factor such as noise, and due to the limitations of the algorithms, there are problems in that it cannot reflect a patient's health condition at the time therein and cannot capture minute changes in electrocardiograms.

A background technology of the present invention is disclosed in Korean Patent Application Publication No. 10-2022-0008447 (published on January 21, 2022).

### Disclosure

### Technical Problem

According to the present invention, there is provided a method of providing electrocardiogram reading service by which primary reading results are acquired from electrocardiogram data received from a client through an artificial intelligence algorithm and final reading results are provided by transmitting the electrocardiogram data and the primary reading results to medical staff.

### Technical Solution

According to an embodiment of the present invention for overcoming the above technical problem, there is provided a method of providing electrocardiogram reading service by using a system for providing electrocardiogram reading service, the method including: receiving an electrocardiogram reading request signal and electrocardiogram data from a client terminal; outputting primary electrocardiogram reading data by inputting the received electrocardiogram data to an electrocardiogram reading model in response to the received electrocardiogram reading request signal; when receiving an additional electrocardiogram request signal from the client, acquiring secondary electrocardiogram reading data by transmitting the primary electrocardiogram reading data to a medical person terminal including a designated medical specialist; calculating a fee in accordance with an electrocardiogram reading service provision range; and transmitting the primary electrocardiogram reading data or secondary electrocardiogram reading data and the calculated fee to the client terminal.

The method may further include collecting read electrocardiogram data, and training the previously constructed electrocardiogram reading model by inputting the collected electrocardiogram data to the electrocardiogram reading model.

Receiving the electrocardiogram data may include receiving unstructured electrocardiogram data including an electrocardiogram picture file, a photo file taken with a portable device or the like, or an electrocardiogram metadata file.

Acquiring the secondary electrocardiogram reading data may include allowing provisional reading to be performed by transmitting the primary electrocardiogram reading data to a terminal of a medical person including a cardiologist, a nurse, a clinical pathologist, or an emergency medical technician at a designated cooperative hospital, and then acquiring final electrocardiogram reading data by transmitting the provisionally read electrocardiogram data to a medical person terminal including a cardiologist who can read the data at the current time.

The method may further include providing information about the service provision range in response to the received electrocardiogram reading request signal, and receiving service request information from the client in accordance with the service provision range.

The information about the service provision range may include information about at least one of the degree of electrocardiogram reading, reading waiting time, the accuracy of reading, a list of medical staff, and the distance between a reading center and the client.

### Advantageous Effects

According to the above-described present invention, the cost and time incurred by unnecessary visits to hospitals attributable to the misreading of electrocardiograms may be reduced, and situations in which a disease is missed, treatment is delayed and death occurs due to misreading may be prevented.

### Description of Drawings

FIG. 1 is a drawing illustrating a system for providing electrocardiogram reading service according to an embodiment of the present invention;
FIG. 2 is a block diagram illustrating the reading server shown in FIG. 1; and
FIG. 3 is a flowchart illustrating a method of providing electrocardiogram reading service using a system for providing electrocardiogram reading service according to an embodiment of the present invention.

### Mode for Invention

Preferred embodiments according to the present invention will be described in detail below with reference to the accompanying drawings. In this process, the thicknesses of lines or sizes of components shown in the drawings may be shown exaggerated for clarity and convenience of description.

Additionally, the terms to be described below are terms defined by taking into consideration the functions thereof in the present invention, and may vary depending on the intention or custom of a user or operator. Accordingly, the definitions of these terms should be made based on the content throughout the present specification.

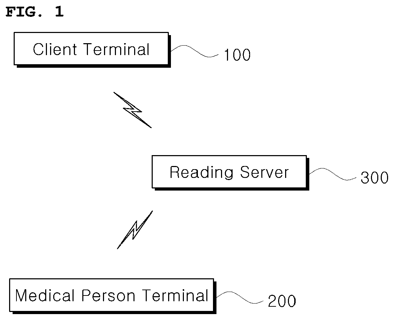

A system for providing electrocardiogram reading service according to an embodiment of the present invention will be described in detail below by using FIG. 1.

FIG. 1 is a drawing illustrating a system for providing electrocardiogram reading service according to an embodiment of the present invention.

As shown in FIG. 1, the system for providing electrocardiogram reading service according to an embodiment of the present invention includes a client terminal 100, a medical person terminal 200, and a reading server 300.

The client terminal 100 transmits an electrocardiogram reading request while executing an app or webpage provided by the reading server 300, and uploads electrocardiogram data to be read.

In this case, the client terminal 100 may receive information about a service provision range for electrocardiogram reading from the reading server 300, and a client may enter an additional electrocardiogram reading request in accordance with the service provision range.

Then, the client terminal 100 receives the results of the electrocardiogram reading according to the completion of the reading.

The medical person terminal 200 is a terminal used by a medical person including a cardiologist, and transmits the results of the reading, input by a medical person including a cardiologist, to the reading server 300, which will be described later. In addition, when the reading is completed, the medical person terminal 200 including a cardiologist requests a fee and receives the requested fee through a virtual account.

Finally, when the reading server 300 receives an electrocardiogram reading request signal from the client terminal 100, it provides information about the reading service provision range and receives corresponding service request information and electrocardiogram data in response thereto.

In addition, the reading server 300 transmits primary electrocardiogram reading data or secondary electrocardiogram reading data to the corresponding client terminal 100 for the input electrocardiogram data according to the input service request information. Furthermore, the reading server 300 calculates a fee for an electrocardiogram service, transmits information about the calculated fee to the client terminal 100, and receives the fee.

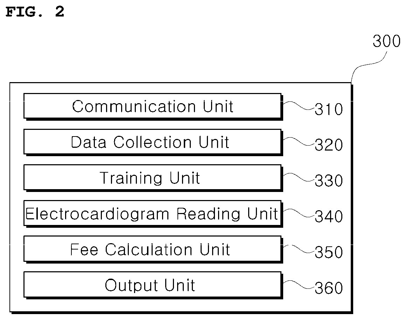

FIG. 2 is a block diagram illustrating the reading server 300 shown in FIG. 1.

As shown in FIG. 2, the reading server 300 according to an embodiment of the present invention includes a communication unit 310, a data collection unit 320, a training unit 330, an electrocardiogram reading unit 340, a fee calculation unit 350, and an output unit 360.

First, the communication unit 310 communicates with the client terminal 100 and the medical person terminal 200 including a cardiologist, and receives an electrocardiogram reading request signal and electrocardiogram data from the client terminal 100. Additionally, the communication unit 310 transmits the received electrocardiogram data to the medical person terminal 200 including a cardiologist, and receives electrocardiogram reading data from the medical person terminal 200 including a cardiologist.

The data collection unit 320 collects the electrocardiogram reading data that has been read by a medical person including a cardiologist. The collected electrocardiogram reading data includes information about at least one of a patient's gender, age, and diagnosed heart disease.

The collected electrocardiogram reading data is transmitted to the training unit 330, and the training unit 330 trains a previously constructed electrocardiogram reading model by inputting electrocardiogram data randomly extracted from the received electrocardiogram reading data to the previously constructed electrocardiogram reading model.

The electrocardiogram reading unit 340 acquires primary electrocardiogram reading data by inputting the received electrocardiogram data to the trained electrocardiogram reading model.

In this case, when receiving a secondary electrocardiogram reading request from the client, the electrocardiogram reading unit 340 transmits primary electrocardiogram reading data to the medical person terminal 200 including a cardiologist, and then receives secondary electrocardiogram reading data from the medical person terminal 200 including a cardiologist.

The fee calculation unit 350 determines whether only the primary electrocardiogram reading data has been requested from the client or whether the secondary electrocardiogram reading data also has been requested from the client. In addition, the calculation unit 350 calculates the fee varying depending to the results of the determination.

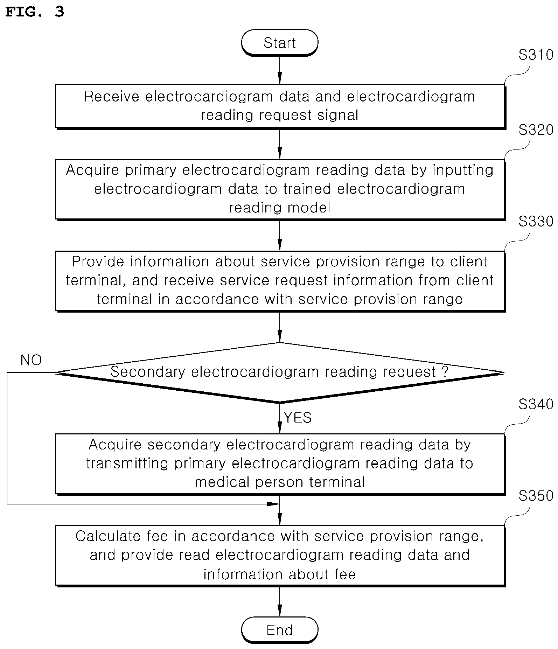

A method of providing electrocardiogram reading service using the system for providing electrocardiogram reading service will be described in more detail below by using FIG. 3.

FIG. 3 is a flowchart illustrating the method of providing electrocardiogram reading service using the system for providing electrocardiogram reading service according to an embodiment of the present invention.

As shown in FIG. 3, a client accesses an app or web page provided by the reading server 300 through the terminal 100. In addition, the client uploads the electrocardiogram data to be read through the terminal 100 and transmits an electrocardiogram reading request signal in step S210.

In this case, the electrocardiogram data may be unstructured electrocardiogram data including an electrocardiogram picture file, a photo file taken with a portable device, etc., or may be an electrocardiogram metadata file.

Next, the reading server 300 acquires primary electrocardiogram reading data by inputting the received electrocardiogram data to a trained electrocardiogram reading model in step S320.

First, the reading server 300 collects the electrocardiogram reading data that has been read. In addition, the training unit 330 of the reading server 300 trains the previously constructed electrocardiogram reading model by inputting the collected electrocardiogram reading data to the electrocardiogram reading model.

When the training of the electrocardiogram reading model is completed, the electrocardiogram reading unit 340 acquires primary electrocardiogram reading data by inputting the received electrocardiogram data to the electrocardiogram reading model.

Next, the reading server 300 additionally provides information about a service provision range to the client terminal 100 and receives service request information from the client terminal 100 in response to the service provision range in step S330.

The information about a service provision range includes information about at least one of the degree of electrocardiogram reading, reading waiting time, reading accuracy, a list of medical staff for reading, and the distance between a reading center and the client.

In this case, the degree of electrocardiogram reading denote whether to perform reading only up to the primary electrocardiogram reading data or up to the secondary electrocardiogram reading data.

When only the primary electrocardiogram reading data is requested by the client terminal 100, the output unit 360 transmits the primary electrocardiogram reading data to the client terminal 100.

In contrast, when the secondary electrocardiogram reading data is requested by the client terminal 100, the electrocardiogram reading unit 340 acquires secondary electrocardiogram reading data by transmitting the primary electrocardiogram reading data to the medical person terminal 200 including a cardiologist in step S340.

The electrocardiogram reading unit 340 extracts the medical person terminal 200 including a cardiologists that can perform electrocardiogram reading at the current time, and transmits an electrocardiogram reading request to the extracted medical person terminal 200 including a cardiologist. In addition, the electrocardiogram reading unit 340 transmits the primary electrocardiogram reading data to the medical person terminal 200 including a cardiologist who has transmitted an electrocardiogram reading acceptance signal.

In the present invention, before the generation of the secondary electrocardiogram reading data using the primary electrocardiogram reading data that is received by a medical person including a cardiologist, provisional reading is performed by transmitting the primary electrocardiogram reading data to the terminal of a medical person including a cardiologist, a nurse, a clinical pathologist, or an emergency medical technician at a designated cooperative hospital.

In addition, when provisional reading is completed, the electrocardiogram reading unit 340 transmits the primary electrocardiogram reading data and the provisionally read electrocardiogram reading data to the cardiologist terminal 200, thereby allowing a medical person including a cardiologist to generate final electrocardiogram reading data.

Finally, the fee calculation unit 350 calculates a fee in accordance with the service provision range in step S350.

The fee for the primary electrocardiogram reading data is free or set to a lower price than that for the secondary reading data. The fee calculation unit 350 calculates a fee based on the preset price.

In addition, the output unit 360 transmits the primary electrocardiogram reading data or secondary electrocardiogram reading data and the calculated fee to the client terminal 100.

The above-described method of providing electrocardiogram reading service using the system for providing electrocardiogram reading service according to the present invention may reduce the cost and time incurred by unnecessary visits to hospitals attributable to the misreading of electrocardiograms, and may prevent situations in which a disease is missed, treatment is delayed and death occurs due to misreading.

While the present invention has been described with reference to the embodiments shown in the drawings, these are merely examples. It will be understood by those skilled in the art that various modifications and other equivalent embodiments may be made therefrom. Therefore, the true technical protection range of the present invention should be determined by the technical spirit of the following patent claims.

## Claims

1. A method of providing electrocardiogram reading service by using a system for providing electrocardiogram reading service, the method comprising:
receiving an electrocardiogram reading request signal and electrocardiogram data from a client terminal;
outputting primary electrocardiogram reading data by inputting the received electrocardiogram data to an electrocardiogram reading model in response to the received electrocardiogram reading request signal;
when receiving an additional electrocardiogram request signal from the client, acquiring secondary electrocardiogram reading data by transmitting the primary electrocardiogram reading data to a medical person terminal including a designated medical specialist;
calculating a fee in accordance with an electrocardiogram reading service provision range; and
transmitting the primary electrocardiogram reading data or secondary electrocardiogram reading data and the calculated fee to the client terminal.

2. The method of claim 1, further comprising collecting read electrocardiogram data, and training the previously constructed electrocardiogram reading model by inputting the collected electrocardiogram data to the electrocardiogram reading model.

3. The method of claim 1, wherein receiving the electrocardiogram data comprises receiving unstructured electrocardiogram data, including an electrocardiogram picture file, a photo file taken with a portable device or the like, or an electrocardiogram metadata file.

4. The method of claim 1, wherein acquiring the secondary electrocardiogram reading data comprises allowing provisional reading to be performed by transmitting the primary electrocardiogram reading data to a terminal of a medical person including a cardiologist, a nurse, a clinical pathologist, or an emergency medical technician at a designated cooperative hospital, and then acquiring final electrocardiogram reading data by transmitting provisionally read electrocardiogram data to a medical person terminal including a cardiologist who can read the data at a current time.

5. The method of claim 1, further comprising providing information about the service provision range in response to the received electrocardiogram reading request signal, and receiving service request information from the client in accordance with the service provision range.

6. The method of claim 5, wherein the information about the service provision range comprises information about at least one of a degree of electrocardiogram reading, reading waiting time, accuracy of reading, a list of medical staff, and a distance between a reading center and the client.
